# EUROPEAN PATENT APPLICATION

(11) **EP 2 336 170 A2**
(43) Date of publication of application: **22.06.2011**
(21) Application number: 10075642.8
(22) Date of filing: 14.12.2006
(51) Int. Cl.: C07K 14/78, C07K 16/28

(54) **Recombinant triplex scaffold-based polypeptides**

(30) Priority: 15.12.2005 US 750746 P
(62) Divisional of application: 06291927.9
(71) Applicant: Industrial Technology Research Institute, Chutung Hsin chu 310 (TW)
(72) Inventor: Chou, Min-Yuan, Taipei County 231 (TW); Fan, Chia-Yu, Hsinchu City 300 (TW); Huang, Chuan-chuan, Taipei County 242 (TW); Li, Hsiu-Chuan, Hsinchu County 308 (TW)
(74) Representative: Lau, Sarah Jane

(57) **Abstract**

Disclosed is a protein complex that includes triple-helix-coil forming fusion polypeptide chains, each having a scaffold domain and a heterologous domain. Also disclosed are related isolated fusion polypeptide, nucleic acids, vectors, host cells, and preparation methods.

## Description

### RELATED APPLICATION

This application claims priority to U.S. Application Serial No. 60/750,746, filed December 15, 2005, the contents of which are incorporated herein by reference.

### BACKGROUND

Protein-based binding reagents have various uses in therapeutic or diagnostic application. Antibodies have proven to be an excellent paradigm for such reagents. Indeed, a number of monoclonal antibodies (mAbs) have been successfully used for treating cancers, infectious diseases, and inflammatory diseases (Adams et al., Nat. Biotechnol. 2005 Sep;23(9):1147-57.).

Both the efficacy and safety of murine mAbs have been enhanced by recombinant DNA technology, including chimerization and humanization. However, humanization of murine mAbs using CDR (complementarity determining region) grafting often results in lower binding affinity than the murine counterparts. Antibody affinity is a key factor in the success of an antibody as a therapeutic agent. An antibody with high affinity allows the antibody to compete effectively with the natural ligand for the targeted receptor, to reduce dosage, toxicity, and cost. Affinity also affects antibody's pharmacokinetics, such as its distribution and excretion within a targeted tissue and the host circulation. A requirement for an effective monoclonal antibody *in vivo* is a strong affinity for the target antigen without nonspecifically binding to unrelated proteins. Multimerization of antigen binding sites has been shown to be an effective means of increasing the overall strength of the binding of an antibody to an antigen which is defined as the antibody avidity (functional affinity) (Miller et al., J Immunol 170:4854-4861,2003; Rheinnecker et al., J Immunol 157:2989-2997, 1996; Shopes, J Immunol 148:2918-2922, 1992; Shuford et al., Science 252:724-727, 1991; Wolff et al., J Immunol 148:2469-2474, 1992). They have increased antitumor activity in vivo (Liu et al., Int Immunopharmacol 6:791-799, 2006; Wolff et al., Cancer Res 53:2560-2565, 1993). Due to the bivalent nature of immunoglobulin G (IgG), conventional and engineered IgG cannot be used for simultaneous binding to more than two different antigens. Thus, there is a need for multivalent or multi-specific protein-based binding reagents.

In some cases, avoiding the effector function such as antibody-dependent cell-mediated cytotoxicity (ADCC) and complement dependent cytotoxicity (CDC) through engineering the Fc region is necessary to reduce the mitogenicity side-effects. For example, the murine anti-human CD3 mAb (Orthoclone OKT3, muromonab-CD3), is a potent immunosuppressive agent targeting the T-cell receptor (TCR)/CD3 complex on human T cells. It has been used during the last two decades to prevent or treat allograft rejection (Cosimi et al., N Engl J Med 305:308-314, 1981; Group, N Engl J Med 313:337-342, 1985; Kung et al., Science 206:347-349, 1979). However, one major drawback to the use of this therapy is the systemic release of cytokines, such as TNF-α, IL-2 and IFN-γ , which result in a series of adverse mitogenic effects, including flu-like symptoms, respiratory distress, neurological symptoms and acute tubular necrosis (Abramowicz et al., Transplantation 47:606-608, 1989; Chatenoud et al., N Engl J Med 320:1420-1421, 1989; Goldman et al., Transplantation 50:158-159, 1990; Toussaint et al., Transplantation 48:524-526, 1989). Since the mitogenic activity of OKT3 and other anti-CD3 mAbs depends upon extensive TCR/CD3 cross-linking via binding to FcR-positive cells (e.g. monocytes), recent efforts have been devoted developing nonmitogenic forms of anti-CD3 antibodies by altering binding to FcR. Thus, there is a need for protein-based binding reagents that have high affinity, low mitogenic effect, and high *in vivo* stability.

### SUMMARY

This invention relates protein complex-based binding reagents that have high affinity, low mitogenic effect, and high *in vivo* stability. The reagents can also be multivalent or multi-specific.

Accordingly, one aspect of this invention features an isolated recombinant protein complex that includes a first fusion polypeptide chain containing a first scaffold domain and a first heterologous domain in-frame fused to one end of the first scaffold domain; a second fusion polypeptide chain containing a second scaffold domain; and a third fusion polypeptide chain containing a third scaffold domain. The first, second, and third scaffold domains are aligned to form a triple helix coil. The first scaffold domain and first heterologous domain are in-frame fused and on the same peptide chain.

The heterologous domain can include the sequence of an enzymatic domain or a fluorescent protein. Examples of a fluorescent protein include GFP and dsRed, as well as their variants. Examples of an enzymatic domain include that of glutathione S-transferase, luciferase, β-galactosidase, and β-lactamase.

The heterologous domain can include a binding domain (e.g., a ligand binding domain, a ligand, a receptor, or a proteoglycan) that binds to a binding partner. A "binding partner" refers to any molecule that has a specific, covalent or non-covalent affinity for a portion of a desired compound (e.g., a protein) of interest. Examples of binding partners include members of antigen/antibody pairs, protein/inhibitor pairs, receptor/ligand pairs (e.g., cell surface or nuclear receptor/ligand pairs), enzyme/substrate pairs (e.g., kinase/substrate pairs), lectin/carbohydrate pairs, oligomeric or heterooligomeric protein pairs, DNA binding protein/DNA binding site pairs, and RNA/protein pairs. Examples of the binding domain also include the sequences of affinity tags, such as a histidine-tag, a myc tag, or a hemagglutin tag.

The above-mentioned first heterologous domain can include one or more complementary-determining regions (CDR) of an immunoglobulin. Accordingly, the heterologous domain can include antigen binding parts of an antibody such as a V_{H} domain and an Fab. In one embodiment, the first heterologous domain contains the sequence of an antigen-binding fragment or a single chain antibody, e.g., that specific for Cluster Designation 3 (CD3) or Epidermal Growth Factor Receptor (EGFR). The first polypeptide chain can further contain a second heterologous domain in-frame fused to the other end of the first scaffold domain. Like the first heterologous domain, the second heterologous domain can also include a domain that binds to a binding partner. The first and second heterologous domains can be identical to or different from each other. They can bind to an identical binding partner or two different binding partners. For example, the first heterologous domain and second heterologous domain can respectively contain the sequences of a first single chain antibody and a second single chain antibody that specifically bind to CD3 and EGFR, respectively.

In the above-described protein complex, the second fusion polypeptide chain can contain a third heterologous domain in-frame fused to one end of the second scaffold domain, a fourth heterologous domain in-frame fused to the other end of the second scaffold domain, or both domains in-frame fused to the two ends. Similarly, the third fusion polypeptide chain can contain a fifth heterologous domain in-frame fused to one end of the third scaffold domain, or a sixth heterologous domain in-frame fused to the other end of the third scaffold domain, or both. Like the first and second heterologous domains described above, each of these four heterologous domains can also include a binding domain that binds to a binding partner. All six heterologous domains can be identical to or different from each other. They therefore can bind to 1, 2, 3, 4, 5, or 6 binding partners. In other words, the protein complex can be mono-, di-, tri-, tetra-, penta-, or hexa-valent.

For the first, second, and third scaffold domains to form a triple helix coil, each of the three scaffold domains contains one or more triple helix repeats, each repeat containing a sequence of the following formula: (X1-X2-X3)n, in which X1 is a Gly residue; X2 and X3 are any amino acid residues, and preferably, the imino acid proline or hydroxyproline; and n is 5 or greater. For example, the first, second, or third scaffold domain can include (GPP)₁₀ or one or more triple helix repeats of human Complement Clq, collectin, or collagen polypeptide chain.

In one example, the above-described first, second, and third fusion polypeptides are substantially identical, having at least 75% (e.g., any number between 75% and 100%, inclusive) sequence identity to one another. A complex formed by three identical fusion polypeptides is a homotrimer. The three fusion polypeptides can be functional equivalents. A "functional equivalent" refers to a polypeptide derivative of a common polypeptide, e.g., a protein having one or more point mutations, insertions, deletions, truncations, a fusion protein, or a combination thereof, and retaining substantially the ability to form a triple helix coil and the activity of the heterologous domain, such as biding to a ligand.

A heterologous polypeptide, nucleic acid, or gene is one that originates from a foreign species, or, if from the same species, is substantially modified from its original form. Two fused domains or sequences are heterologous to each other if they are not adjacent to each other in a naturally occurring protein or nucleic acid. For example, a polypeptide sequence that is not a part of naturally occurring human minicollagen (type XXI), collectin family protein, or complement lq (Clq) is heterologous to a domain in the human protein.

This invention also features an isolated recombinant fusion polypeptide (e.g., each of the above-mentioned three fusion polypeptides) that contains (i) a scaffold domain for forming a triple helix coil and (ii) a first heterologous domain in-frame fused to one end of the scaffold domain or a second heterologous domain in-frame fused to the other end of the scaffold domain. The scaffold domain can contain one or more of the above-mentioned triple helix repeats, e.g., that of human Clq or collagen polypeptide chain. The heterologous domain can include one of the binding domain mentioned above, and can be obtained by various art-recognized methods, such as phage display screening.

An isolated polypeptide or protein complex refers to a polypeptide or a protein complex substantially free from naturally associated molecules, i.e., it is at least 75% (i.e., any number between 75% and 100%, inclusive) pure by dry weight. Purity can be measured by any appropriate standard method, for example, by column chromatography, polyacrylamide gel electrophoresis, or HPLC analysis. An isolated polypeptide or protein complex of the invention can be purified from a natural source, produced by recombinant DNA techniques.

The invention also features an isolated nucleic acid that contains a sequence encoding the just-mentioned fusion polypeptide or a complement of the sequence. A nucleic acid refers to a DNA molecule (e.g., a cDNA or genomic DNA), an RNA molecule (e.g., an mRNA), or a DNA or RNA analog. A DNA or RNA analog can be synthesized from nucleotide analogs. The nucleic acid molecule can be single-stranded or double-stranded, but preferably is double-stranded DNA. An "isolated nucleic acid" is a nucleic acid the structure of which is not identical to that of any naturally occurring nucleic acid or to that of any fragment of a naturally occurring genomic nucleic acid. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. The nucleic acid described above can be used to express the polypeptide of this invention. For this purpose, one can operatively link the nucleic acid to suitable regulatory sequences to generate an expression vector.

A vector refers to a nucleic acid molecule capable of transporting another nucleic acid to which it has been linked. The vector can be capable of autonomous replication or integrate into a host DNA. Examples of the vector include a plasmid, cosmid, or viral vector. The vector of this invention includes a nucleic acid in a form suitable for expression of the nucleic acid in a host cell. Preferably the vector includes one or more regulatory sequences operatively linked to the nucleic acid sequence to be expressed. A "regulatory sequence" includes promoters, enhancers, and other expression control elements (e.g., polyadenylation signals). Regulatory sequences include those that direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vector can be introduced into host cells to produce the polypeptide of this invention. Also within the scope of this invention is a host cell that contains the above-described nucleic acid. Examples include *E*. *coli* cells, insect cells (e.g., using *Drosophila* S2 cells or baculovirus cells), yeast cells, or mammalian cells (e.g., mouse myeloma NS0 cell). See e.g., Goeddel, (1990) Gene Expression Technology: Methods in Enzymology 185, Academic Press, San Diego, CA.

To produce a fusion polypeptide of this invention, one can culture a host cell in a medium under conditions permitting expression of the polypeptide encoded by a nucleic acid of this invention, and purify the polypeptide from the cultured cell or the medium of the cell. Alternatively, the nucleic acid of this invention can be transcribed and translated in vitro, for example, using T7 promoter regulatory sequences and T7 polymerase.

To produce a protein complex of this invention, one can culture a host cell containing a first, second, and third nucleic acids respectively encoding the above-mentioned first, second, and third fusion polypeptides in a medium under a condition permitting expression of polypeptides encoded by the three nucleic acids and formation of a triple helix coil between the expressed polypeptides, and purifying the protein complex from the cultured cell or the medium of the cell. Preferably, the host cell is a eukaryotic cell containing an enzymatic activity that hydroxylates a proline residue.

The details of one or more embodiments of the invention are set fourth in the accompanying drawing and the description below. Other features, objects, and advantages of the invention will be apparent from the description and drawing, and from the claims.

### DESCRIPTION OF DRAWINGS

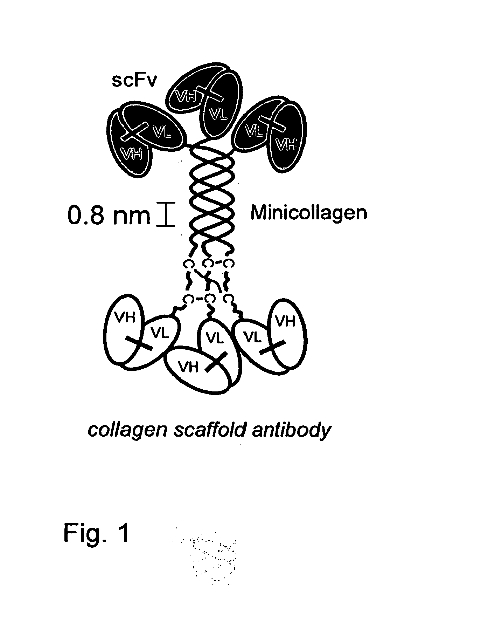

FIG. 1 is a diagram showing a protein complex that has a minicollagen triple helix coil scaffold derived from human type XXI collagen. The six termini of the triple helix coil are in-frame fused to six Fv fragments that have V_{L} and V_{H} domains of single chain antibodies (scFv), respectively.

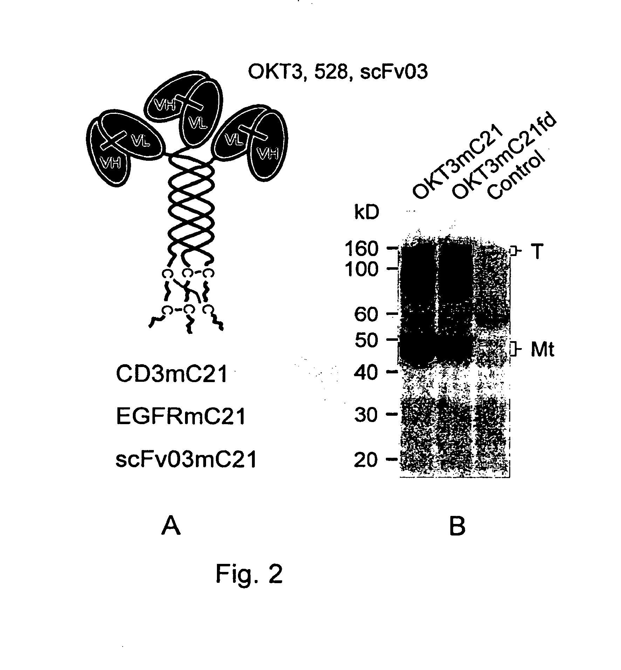

FIGs. 2A and 2B are respectively (a) a diagram showing a protein complex that has a triple helix coil scaffold the three N termini of which are respectively in-frame fused to three single chain antibodies OKT3 (anti-CD3), 528 (anti-EGFR), and erb_scFv (anti-EGFR) and (b) a photo showing Western Blot results of the protein complex. The culture media from the stably transfected *Drosophila* S2 cells was electrophoresed on a SDS-PAGE under non-reducing conditions and then immunoblotted with a monoclonal antibody to the C-terminal of type XXI collagen, 3E2. *T*: interchain disulfide-bonded trimers; *Mt*: monomers containing intrachain disulfide-bonded trimers.

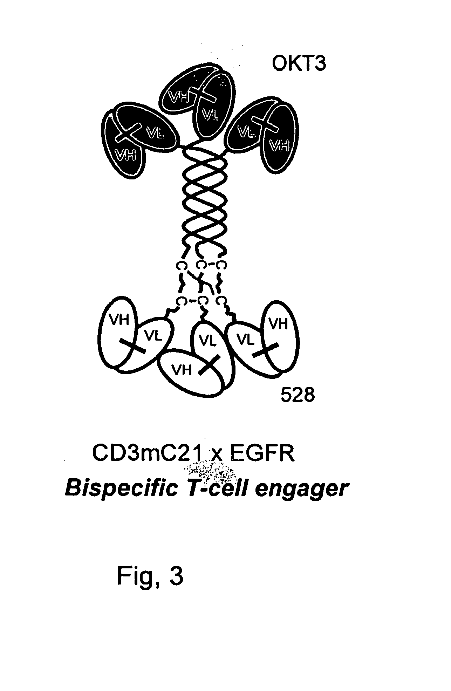

FIG. 3 is a diagram showing a protein complex that has a triple helix coil scaffold. The three N termini of the triple helix coil are in-frame fused to three OKT3 single chain antibodies; the three C termini of the triple helix coil are in-frame fused to three 528 single chain antibodies.

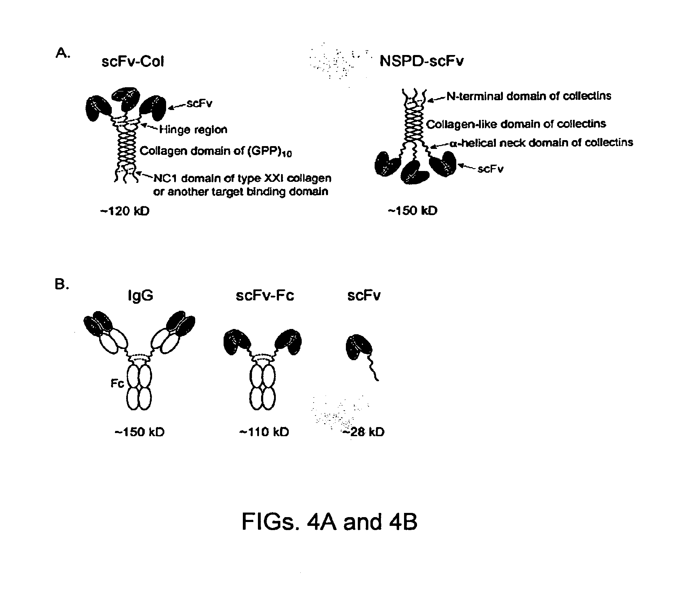

FIGs. 4A and 4B are schematic representations of different formats of antibodies: (A) collagen scaffold antibodies: scFv-Col (left panel), containing an amino-terminal scFv, a hinge region of human IgG, a collageneous domain (GPP)₁₀, and a carboxyl-terminal NCl domain of type XXI collagen; NSPD-scFv (right panel), containing an amino-terminal portion of surfactant protein D (SPD) collectin and an scFv at the carboxyl-terminus. (B) from left to right: immunoglobulin G (IgG), chimeric (scFv-Fc), and single-chain antibody (scFv) with their respective approximate molecular masses. Grey areas represent the V_{H} and V_{L} fragments; dashed lines: inter-chain disulfide-bonds.

### DETAILED DESCRIPTION

The present invention is based, at least in part, on the unexpected findings that a heterologous protein binding domain in-frame fused to a human triple helix coil scaffold domain retains the binding activity and that the resulting fusion polypeptide forms a triple helix coil. Shown in FIG.1 is one example of a protein complex of this invention. As shown in this figure, the six termini of a triple helix coil protein complex are respectively in-frame fused to six heterologous protein binding domains, i.e., Fv fragments of single chain antibodies (scFv).

A protein complex of this invention enjoys advantages over conventional antibodies. On the one hand, when two or more of the six domains are identical to each other, the protein complex can have 2-6 binding domains that are specific for one binding partner (e.g., antigen) in comparison with a conventional antibody, which has only two such domains. In other words, unlike a conventional antibody, which is only divalent for an antigen, the protein complex can be di-, tri-, tetra-, penta-, or hexa-valent. As a result, it can be made to have various affinities that are higher than a conventional antibody. Because of the higher affinities, smaller amounts of the protein complex and shorter incubation duration are needed than a conventional antibody to achieve the desired goals. For example, therapeutic effects, thereby lowering treatment costs and minimizing side effects (e.g., unwanted immune responses). On the other hand, when two or more of the six domains are different from each other, a protein complex of this invention can have 2-6 binding domains that are specific for 2-6 different binding partners. Unifying multiple binding partner sites of different specificities into one unit, it has the ability to bring together multiple binding partners and therefore have desirable uses in therapy, tissue reconstruction, and assembly of active protein machinery (e.g., a multi-subunit enzyme) at the nanometer level.

For *in vivo* use in a human, a protein complex of this invention is preferably of a human origin. For example, it can include a humanized single chain antibody sequence in-frame fused to helix coil scaffold of human origin, such as that of human Clq, collectin family proteins, or collagen polypeptide chain. As both human Clq and collagen are fairly stable in the blood, the protein complex is more stable than conventional antibodies.

Collagen is the most abundant protein present in mammals. It is an extracellular matrix protein containing the repeating triplet sequence Gly-X1-X2 and the presence of such triplets allows three collagen polypeptide chains (α-chains) to fold into a triple-helical conformation. The X2 position amino acid in the triplet sequence Gly-X1-X2 is frequently proline, which is often 4-hydroxylated by post-translational modification of the collagen polypeptide chain in order to stabilize the triple-helical structure of collagen. In the absence of proline hydroxylation, the essential triple helical conformation of collagen is thermally unstable at below physiological temperatures (Berg and Prockop, Biochem Biophys Res Commun 52:115-120, 1973; Rosenbloom et al., Arch Biochem Biophys 158:478-484, 1973). Many collagen-like proteins with collagenous domains are present in human serum and serve as innate immune system in protection from infectious organisms. These include complement protein Clq, collectin family proteins - mannose binding lectin (MBL), surfactant proteins A and D (SP-A and SP-D). A common structural feature among these collagen-like proteins is that all of them are in multimeric protein units by triple helical assembly of their collagenous domains, followed by stacking or interchain disulfide crosslinking of the trimeric molecules. Consequently, the functional affinity of the binding domain of these "defense collagen" molecules is increased greatly through multimerization.

A protein complex or polypeptide of the invention can be obtained by recombinant technology. One can introduce into suitable host cells nucleic acids encoding polypeptides of the complex and express the polypeptides under a condition permitting expression of polypeptides encoded by the nucleic acids and formation of a triple helix coil between the polypeptides. To facilitate formation of the triple helix coil scaffold, one can co-express in the host cells prolyl 4-hydroxylase (P4HA), a key enzyme in the biosynthesis of collagen.

A heterologous protein domain can contain an antibody or a fragment thereof (e.g., an antigen-binding fragment thereof). The term "antibody" as used herein refers to an immunoglobulin molecule or immunologically active portion thereof, i.e., an antigen-binding portion. It refers to a protein comprising at least one, and preferably two, heavy (H) chain variable regions (V_{H}), and at least one and preferably two light (L) chain variable regions (V_{L}). The V_{H} and V_{L} regions can be further subdivided into regions of hypervariability, termed "complementarity determining regions" ("CDR"), interspersed with regions that are more conserved, termed "framework regions" (FR). The extent of the framework region and CDR's has been precisely defined (see, Kabat et al. (1991) Sequences of Proteins of Immunological Interest, Fifth Edition, U.S. Department of Health and Human Services, NIH Publication No. 91-3242, and Chothia et al. (1987) J. Mol. Biol. 196:901-917, which are incorporated herein by reference). Each V_{H} and V_{L} is composed of three CDR's and four FRs, arranged from amino-terminus to carboxy-terminus in the following order: FR1, CDR1, FR2, CDR2, FR3, CDR3, FR4. The antibody can further include a heavy and light chain constant region, to thereby form a heavy and light immunoglobulin chain, respectively. The heavy chain constant region is comprised of three domains, CH1, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

As used herein, the term "immunoglobulin" refers to a protein consisting of one or more polypeptides substantially encoded by immunoglobulin genes. The recognized human immunoglobulin genes include the kappa, lambda, alpha (IgA1 and IgA2), gamma (IgG1, IgG2, IgG3, IgG4), delta, epsilon and mu constant region genes, as well as the myriad immunoglobulin variable region genes. Full-length immunoglobulin "light chains" (about 25 KDa or 214 amino acids) are encoded by a variable region gene at the NH2-terminus (about 110 amino acids) and a kappa or lambda constant region gene at the COOH--terminus. Full-length immunoglobulin "heavy chains" (about 50 KDa or 446 amino acids), are similarly encoded by a variable region gene (about 116 amino acids) and one of the other aforementioned constant region genes, e.g., gamma (encoding about 330 amino acids).

The term "antigen-binding fragment" of an antibody (or "antibody portion," or "fragment"), as used herein, refers to one or more fragments of a full-length antibody that retain the ability to specifically bind to the antigen, e.g., EGFR or CD3 polypeptide or fragment thereof. Examples of antigen-binding fragments of the antibody include, but are not limited to: (i) a Fab fragment, a monovalent fragment consisting of the V_{L}, V_{H}, C_{L} and C_{H1} domains; (ii) a F(ab')₂ fragment, a bivalent fragment comprising two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546), which consists of a V_{H} domain; (vi) an isolated complementarity determining region (CDR), and (vii) V_{L} or V_{H} domains. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see *e.g.*, Bird et al. (1988) Science 242:423-426; and Huston et al. (1988) Proc. Natl. Acad. Sci. USA 85:5879-5883). Such single chain antibodies are also encompassed within the term "antigen-binding fragment" of an antibody. These antibody fragments can be obtained using conventional techniques known to those with skill in the art, and the fragments are screened for utility in the same manner as are intact antibodies.

Suitable antibody can be a monoclonal antibody. In other embodiments, the antibody can be recombinantly produced, e.g., produced by phage display or by combinatorial methods. Phage display and combinatorial methods for generating antibodies are known in the art (see e.g., Ladner et al. U.S. Patent No. 5,223,409; Kang et al. International Publication No. WO 92/186119; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982, the contents of all of which are incorporated by reference herein).

In one embodiment, the antibody is a fully human antibody (e.g., an antibody made in a mouse which has been genetically engineered to produce an antibody from a human immunoglobulin sequence), or a non-human antibody, e.g., a rodent (mouse or rat), goat, primate (e.g., monkey), camel antibody. Preferably, the non-human antibody is a rodent (mouse or rat antibody). Methods of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L.L. et al. 1994 Nature Genet. 7:13-21; Morrison et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326).

An antibody can be one in which the variable region, or a portion thereof, e.g., the CDR's, are generated in a non-human organism, e.g., a rat or mouse. Chimeric, CDR-grafted, and humanized antibodies can be used. Antibodies generated in a non-human organism, e.g., a rat or mouse, and then modified, e.g., in the variable framework or constant region, to decrease antigenicity in a human are within the invention.

Chimeric antibodies can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted (see Robinson et al., International Patent Publication PCT/US86/02269; Akira, *et al.,* European Patent Application 184,187; Taniguchi, M., European Patent Application 171,496; Morrison *et al.,* European Patent Application 173,494; Neuberger et al., International Application WO 86/01533; Cabilly et al. U.S. Patent No. 4,816,567; Cabilly *et al.,* European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. et al (1985) Nature 314:446-449; and Shaw et al., 1988, J. Natl Cancer Inst. 80:1553-1559).

A humanized or CDR-grafted antibody will have at least one or two but generally all three recipient CDR's (of heavy and or light immuoglobulin chains) replaced with a donor CDR. The antibody may be replaced with at least a portion of a non-human CDR or only some of the CDR's may be replaced with non-human CDR's. It is only necessary to replace the number of CDR's required for binding of the humanized antibody or a fragment thereof. Preferably, the donor will be a rodent antibody, e.g., a rat or mouse antibody, and the recipient will be a human framework or a human consensus framework. Typically, the immunoglobulin providing the CDR's is called the "donor" and the immunoglobulin providing the framework is called the "acceptor." In one embodiment, the donor immunoglobulin is a non-human (e.g., rodent). The acceptor framework is a naturally-occurring (e.g., a human) framework or a consensus framework, or a sequence about 85% or higher, preferably 90%, 95%, 99% or higher identical thereto. As used herein, the term "consensus sequence" refers to the sequence formed from the most frequently occurring amino acids (or nucleotides) in a family of related sequences (See e.g., Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody can be humanized by methods known in the art. Humanized antibodies can be generated by replacing sequences of the Fv variable region which are not directly involved in antigen binding with equivalent sequences from human Fv variable regions. General methods for generating humanized antibodies are provided by Morrison, S. L., 1985, Science 229:1202-1207, by Oi et al., 1986, BioTechniques 4:214, and by Queen et al. US 5,585,089, US 5,693,761 and US 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a polypeptide of interest or fragment thereof. The recombinant DNA encoding the humanized antibody, or fragment thereof, can then be cloned into an appropriate expression vector.

Also can be fused to the scaffold are humanized antibodies in which specific amino acids have been substituted, deleted or added. Preferred humanized antibodies have amino acid substitutions in the framework region, such as to improve binding to an antigen. For example, a humanized antibody will have framework residues identical to the donor framework residue or to another amino acid other than the recipient framework residue. To generate such antibodies, a selected, small number of acceptor framework residues of the humanized immunoglobulin chain can be replaced by the corresponding donor amino acids. Preferred locations of the substitutions include amino acid residues adjacent to the CDR, or which are capable of interacting with a CDR. Criteria for selecting amino acids from the donor are described in US 5,585,089, the contents of which are hereby incorporated by reference. Other techniques for humanizing antibodies are described in Padlan et al. EP 519596 A1.

The invention also includes a nucleic acid which encodes a fusion polypeptide that forms a protein complex of this invention. The nucleic acid can be screened from phage display library or isolated (e.g., by RT-PCR) from cell lines expressing the above-described suitable antibodies or antibody derivatives. The nucleic acid can be functionally ligated into an expression vector. Cells transformed with the nucleic acid or vector can be used to produce the fusion polypeptide or protein complex of this invention. Cells useful for producing an antibody include insect cells and mammalian cells (e.g. CHO or lymphatic cells).

A protein complex of this invention may be conjugated to a therapeutic moiety such as a cytotoxin, a therapeutic agent or a radioactive ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, *e.g*., maytansinol (see US Patent No. 5,208,020), CC-1065 (see US Patent Nos. 5,475,092, 5,585,499, 5,846,545) and analogs or homologs thereof. Therapeutic agents include, but are not limited to, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, CC-1065, melphalan, carmustine (BSNU) and lomustine (CCNU), cyclothosphamide, busulfan, dibromomannitol, streptozotocin, mitomycin C, and cisdichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g*., vincristine, vinblastine, taxol and maytansinoids). Radioactive ions include, but are not limited to iodine, yttrium and praseodymium.

The conjugates can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

The above-described protein complexes and conjugates, basing on the specificity of the heterologous binding domains, can be used for treating various disorders, including cancers, inflammation diseases, metabolism diseases, fibrosis diseases, and cardiovascular diseases. The invention therefore features a method of treating such a disorder, e.g., by administering to a subject in need thereof an effective amount of an protein complex of the invention. Subjects to be treated can be identified as having, or being at risk for acquiring, a condition characterized by the disorder. This method can be performed alone or in conjunction with other drugs or therapy.

Because of the multi-specific feature of a protein complex of this invention, one can use it to bridge molecules or cells that are normally are not associated with each other. This feature is particularly useful for cell-based therapies. In one example, one heterologous domain in the protein complex is capable of activating cytotoxic cells (e.g., cytotoxic T cells) by specifically binding to an effector antigen on the cytotoxic cells, while another heterologous domain specifically binds to a target antigen on a pathogen cell or a malignant cell to be destructed. In this way, the protein complex can treated a disorder caused by the pathogen or malignant cells.

Activation of the cytotoxic T cell may occur via binding of the CD3 antigen as an effector antigen on the surface of the cytotoxic T cell by a protein complex of the invention. Other lymphoid cell-associated effector antigens include the human CD 16 antigen, NKG2D antigen, NKp46 antigen, CD2 antigen, CD28 antigen, CD25 antigen, CD64 antigen, and CD89 antigen. Binding to these effector antigens leads to activation of effector cells such as monocytes, neutrophilic granulocytes, and dendritic cells. These activated cells then exert a cytotoxic or an apoptotic effect on target cells.

The target antigen is an antigen which is uniquely expressed on a target cell associate with a disease condition, but which is not expressed, expressed at a low level, or non-accessible in a healthy condition. Examples of such target antigens associates with malignant cells include EpCAM, CCR5, CD19, HER-2 neu, HER-3, HER-4, EGFR, PSMA, CEA, MUC-1 (mucin), MUC2, MUC3, MUC4, MUC5.sub.AC, MUC5.sub.B, MUC7, beta.hCG, Lewis-Y, CD20, CD33, CD30, ganglioside GD3, 9-0-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, and CD63.

The term "treating" is defined as administration of a composition to a subject with the purpose to cure, alleviate, relieve, remedy, prevent, or ameliorate a disorder, the symptom of the disorder, the disease state secondary to the disorder, or the predisposition toward the disorder. An "effective amount" is an amount of the composition that is capable of producing a medically desirable result, e.g., as described above, in a treated subject.

In one in vivo approach, a therapeutic composition (e.g., a composition containing a protein complex the invention) is administered to a subject. Generally, the complex is suspended in a pharmaceutically-acceptable carrier (e.g., physiological saline) and administered orally or by intravenous infusion, or injected or implanted subcutaneously, intramuscularly, intrathecally, intraperitoneally, intrarectally, intravaginally, intranasally, intragastrically, intratracheally, or intrapulmonarily.

The dosage required depends on the choice of the route of administration; the nature of the formulation; the nature of the subject's illness; the subject's size, weight, surface area, age, and sex; other drugs being administered; and the judgment of the attending physician. Suitable dosages are in the range of 0.01-100.0 mg/kg. Variations in the needed dosage are to be expected in view of the variety of compositions available and the different efficiencies of various routes of administration. For example, oral administration would be expected to require higher dosages than administration by intravenous injection. Variations in these dosage levels can be adjusted using standard empirical routines for optimization as is well understood in the art. Encapsulation of the composition in a suitable delivery vehicle (e.g., polymeric microparticles or implantable devices) may increase the efficiency of delivery, particularly for oral delivery.

Also within the scope of this invention is a pharmaceutical composition that contains a pharmaceutically acceptable carrier and an effective amount of a protein complex of the invention. The pharmaceutical composition can be used to treat the disorders listed above. The pharmaceutically acceptable carrier includes a solvent, a dispersion medium, a coating, an antibacterial and antifungal agent, and an isotonic and absorption delaying agent. The pharmaceutical composition can be formulated into dosage forms for different administration routes utilizing conventional methods.

The efficacy of a composition of this invention can be evaluated both *in vitro* and *in vivo.* For *in vivo* studies, the composition can be injected into an animal (e.g., a mouse model) and its therapeutic effects are then accessed. Based on the results, an appropriate dosage range and administration route can be determined.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### Example 1

M13 phage display libraries were screened to identify human single-chain variable fragment (scFv) that specifically binds to EGFR. A numbers of clones were identified. After confirmation by Western Blotting and ELISA, one clone, erb_scFv, was selected for further experiment. The cDNA encoding erb_scFv was obtained and ligated into an expression vector by a standard method. Listed below are the polypeptide sequence of erb_scFv (SEQ ID NO:1) and the nucleotide sequence encoding it (SEQ ID NO:2).

SEQ ID NO: 1:
MetAlaGluValGlnLeuLeuGluSerGlyGlyGlyLeuValGlnProGlyGlySerLeu
ArgLeuSerCysAlaAlaSerGlyPheThrPheSerSerTyrAlaMetSerTrpValArg
GlnAlaProGlyLysGlyLeuGluTrpValSerAspIleGlyAlaSerGlySerAlaThr
SerTyrAlaAspSerValLysGlyArgPheThrIleSerArgAspAsnSerLysAsnThr
LeuTyrLeuGlnMetAsnSerLeuArgAlaGluAspThrAlaValTyrTyrCysAlaLys
SerThrThrThrPheAspTyrTrpGlyGlnGlyThrLeuValThrValSerSerGlyGly
GlyGlySerGlyGlyGlyGlySerGlyGlyGlyGlySerThrAspIleGlnMetThrGln
SerProSerSerLeuSerAlaSerValGlyAspArgValThrIleThrCysArgAlaSer
GlnSerIleSerSerTyrLeuAsnTrpTyrGlnGlnLysProGlyLysAlaProLysLeu
LeuIleTyrAspAlaSerAlaLeuGlnSerGlyValProSerArgPheSerGlySerGly
SerGlyThrAspPheThrLeuThrIleSerSerLeuGlnProGluAspPheAlaThrTyr
TyrCysGlnGlnTyrAlaAspTyrProThrThrPheGlyGlnGlyThrLysValGluIle
LysArg

SEQ ID NO: 2
ATGGCCGAGGTGCAGCTGTTGGAGTCTGGGGGAGGCTTGGTACAGCCTGGGGGGTCCCTG
AGACTCTCCTGTGCAGCCTCTGGATTCACCTTTAGCAGCTATGCCATGAGCTGGGTCCGC
CAGGCTCCAGGGAAGGGGCTGGAGTGGGTCTCAGATATTGGTGCTTCTGGTTCTGCTACA
TCTTACGCAGACTCCGTGAAGGGCCGGTTCACCATCTCCAGAGACAATTCCAAGAACACG

CTGTATCTGCAAATGAACAGCCTGAGAGCCGAGGACACGGCCGTATATTACTGTGCGAAA
TCTACTACTACTTTTGACTACTGGGGCCAGGGAACCCTGGTCACCGTCTCGAGCGGTGGA
GGCGGTTCAGGCGGAGGTGGCAGCGGCGGTGGCGGGTCGACGGACATCCAGATGACCCAG
TCTCCATCCTCCCTGTCTGCATCTGTAGGAGACAGAGTCACCATCACTTGCCGGGCAAGT
CAGAGCATTAGCAGCTATTTAAATTGGTATCAGCAGAAACCAGGGAAAGCCCCTAAGCTC
CTGATCTATGATGCATCCGCTTTGCAAAGTGGGGTCCCATCAAGGTTCAGTGGCAGTGGA
TCTGGGACAGATTTCACTCTCACCATCAGCAGTCTGCAACCTGAAGATTTTGCAACTTAC
TACTGTCAACAGTATGCTGATTATCCTACTACGTTCGGCCAAGGGACCAAGGTGGAAATC
AAACGG

The expression vector was then expressed in insect cell line *Drosophila S2*. The anti-EGFR erb_scFv was purified and subjected to Western Blot analysis and ELISA to confirm its specificity against EGFR.

RT-PCR was conducted to obtain from hybridoma cell lines cDNAs encoding the heavy chain variable region (V_{H}) and light chain variable region (V_{L}) of anti-CD3 monoclonal antibody OKT3. Then, the two cDNAs were ligated to generate a fusion sequence that encodes a fusion protein of V_{H}-V_{L} of OKT3. Listed below are the polypeptide sequence of this fusion protein (SEQ ID NO: 3) and the cDNA sequence encoding it (SEQ ID NO: 4).

SEQ ID NO: 3
ValGlnLeuGlnGlnSerGlyAlaGluLeuAlaArgProGlyAlaSerValLysMetSerC
ysLysAlaSerGlyTyrThrPheThrArgTyrThrMetHisTrpValLysGlnArgProGl
yGlnGlyLeuGluTrpIleGlyTyrIleAsnProSerArgGlyTyrThrAsnTyrAsnGln
LysPheLysAspLysAlaThrLeuThrThrAspLysSerSerSerThrAlaTyrMetGlnL
euSerSerLeuThrSerGluAspSerAlaValTyrTyrCysAlaArgTyrTyrAspAspHi
sTyrCysLeuAspTyrTrpGlyGlnGlyThrThrValThrValSerSerGlyGlyGlyGly
SerGlyGlyGlyGlySerGlyGlyGlyGlySerAspIleValLeuThrGlnSerProAlaI
leMetSerAlaSerProGlyGluLysValThrMetThrCysSerAlaSerSerSerValSe
rTyrMetAsnTrpTyrGlnGlnLysSerGlyThrSerProLysArgTrpIleTyrAspThr
SerLysLeuAlaSerGlyValProAlaHisPheArgGlySerGlySerGlyThrSerTyrS
erLeuThrIleSerGlyMetGluAlaGluAspAlaAlaThrTyrTyrCysGlnGlnTrpSe
rSerAsnProPheThrPheGlySerGlyThrLysLeuGluLeuLysArg

SEQ ID NO: 4
GTCCAGCTGCAGCAGTCAGGGGCTGAACTGGCAAGACCTGGGGCCTCAGTGAAGATGTCC
TGCAAGGCTTCTGGCTACACCTTTACTAGGTACACGATGCACTGGGTAAAACAGAGGCCT
GGACAGGGTCTGGAATGGATTGGATACATTAATCCTAGCCGTGGTTATACTAATTACAAT
CAGAAGTTCAAGGACAAGGCCACATTGACTACAGACAAATCCTCCAGCACAGCCTACATG
CAACTGAGCAGCCTGACATCTGAGGACTCTGCAGTCTATTACTGTGCAAGATATTATGAT
GATCATTACTGCCTTGACTACTGGGGCCAAGGCACCACGGTCACCGTCTCCTCAGGTGGA
GGCGGTTCAGGCGGAGGTGGCTCTGGCGGTGGCGGATCGGACATTGTGCTAACCCAGTCT
CCAGCAATCATGTCTGCATCTCCAGGGGAGAAGGTCACCATGACCTGCAGTGCCAGCTCA

AGTGTAAGTTACATGAACTGGTACCAGCAGAAGTCAGGCACCTCCCCCAAAAGATGGATT
TATGACACATCCAAACTGGCTTCTGGAGTCCCTGCTCACTTCAGGGGCAGTGGGTCTGGG
ACCTCTTACTCTCTCACAATCAGCGGCATGGAGGCTGAAGATGCTGCCACTTATTACTGC
CAGCAGTGGAGTAGTAACCCATTCACGTTCGGCTCGGGGACCAAGCTGGAGCTGAAACGA

The same procedures were performed to obtain cDNAs encoding V_{H} and V_{L} of anti-EGFR monoclonal antibody 528 and a fusion sequence that encodes a fusion protein of V_{H}-V_{L} of the anti-EGFR 528. The 528 monoclonal antibody binds to EGFR on cellular membranes, e.g., on human epidermoid carcinoma A431 cells. The polypeptide sequence of this 528 single-chain antibody (SEQ ID NO: 5) and the cDNA sequence encoding it (SEQ ID NO:6) are listed below.

SEQ ID NO: 5
ValLysLeuGlnGluSerGlySerGluMetAlaArgProGlyAlaSerValLysLeuPro
CysLysAlaSerGlyAspThrPheThrSerTyrTrpMetHisTrpValLysGlnArgHis
GlyHisGlyProGluTrpIleGlyAsnIleTyrProGlySerGlyGlyThrAsnTyrAla
GluLysPheLysAsnLysValThrLeuThrValAspArgSerSerArgThrValTyrMet
HisLeuSerArgLeuThrSerGluAspPheAlaValTyrTyrCysThrArgSerGlyGly
ProTyrPhePheAspTyrTrpGlyGlnGlyThrThrValThrValSerSerGlyGlyGly
GlySerGlyGlyGlyGlySerGlyGlyGlyGlySerMetThrGlnThrProLeuSerLeu
ProValSerLeuGlyAspGlnAlaSerIleSerCysArgSerSerGlnAsnIleValHis
AsnAsnGlyIleThrTyrLeuGluTrpTyrLeuGlnArgProGlyGlnSerProLysLeu
LeuIleTyrLysValSerAspArgPheSerGlyValProAspArgPheSerGlySerGly
SerGlyThrAspPheThrLeuLysIleSerArgValGluAlaGluAspLeuGlyIleTyr
TyrCysPheGlnGlySerHisHisProProThrPheGlyGlyGlyThrLysLeuGlu

SEQ ID NO: 6
GTCAAGCTGCAGGAGTCAGGGTCTGAGATGGCGAGGCCTGGAGCTTCAGTGAAGCTGCCC
TGCAAGGCTTCTGGCGACACATTCACCAGTTACTGGATGCACTGGGTGAAGCAGAGGCAT
GGACATGGCCCTGAGTGGATCGGAAATATTTATCCAGGTAGTGGTGGTACTAACTACGCT
GAGAAGTTCAAGAACAAGGTCACTCTGACTGTAGACAGGTCCTCCCGCACAGTCTACATG
CACCTCAGCAGGCTGACATCTGAGGACTTTGCGGTCTATTATTGTACAAGATCGGGGGGT
CCCTACTTCTTTGACTACTGGGGCCAAGGGACCACGGTCACCGTCTCCTCAGGTGGAGGC
GGTTCAGGCGGAGGTGGCTCTGGCGGTGGCGGATCGATGACCCAAACTCCACTCTCCCTG
CCTGTCAGTCTTGGAGATCAAGCCTCCATCTCTTGCAGATCTAGTCAGAACATTGTACAT
AATAATGGAATCACCTATTTAGAATGGTACCTGCAAAGGCCAGGCCAGTCTCCAAAGCTC
CTGATCTACAAAGTTTCCGACCGATTTTCTGGGGTCCCAGACAGGTTCAGTGGCAGTGGA
TCAGGGACAGATTTCACACTCAAGATCAGCAGAGTAGAGGCTGAGGATCTGGGAATTTAT
TACTGCTTTCAAGGTTCACATCATCCTCCCACGTTCGGCGGGGGGACCAAGCTGGAA

The cDNAs encoding the above-described anti-EGFR scFv03, OKT3 V_{H}-V_{L}, and anti-EGFR 528V_{H}-V_{L} were respectively in-frame fused to the human minicollagen XXI cDNA containing a short hinge sequence at the 5' end and a histidine tag sequence at the 3' end.. Shown below are human minicollagen XXI polypeptide and cDNA sequences (SEQ ID NO: 7 and 8, respectively).

SEQ ID NO: 7:
GlyGlyArgGluProLysSerCysAspLysThrHisThrCysProProCysProArgSer
IleProGlyProProGlyProIleGlyProGluGlyProArgGlyLeuProGlyLeuPro
GlyArgAspGlyValProGlyLeuValGlyValProGlyArgProGlyValArgGlyLeu
LysGlyLeuProGlyArgAsnGlyGluLysGlySerGlnGlyPheGlyTyrProGlyGlu
GlnGlyProProGlyProProGlyProGluGlyProProGlyIleSerLysGluGlyPro
ProGlyAspProGlyLeuProGlyLysAspGlyAspHisGlyLysProGlyIleGlnGly
GlnProGlyProProGlyIleCysAspProSerLeuCysPheSerValIleAlaArgArg
AspProPheArgLysGlyProAsnTyrSerLeuAspAspSerSerHisHisHisHisHis
HisSerSerGly
(note: Pro= Proline or Hydroxyproline)

SEQ ID NO: 8:
GGCGGCCGCGAGCCCAAATCTTGTGACAAAACTCACACATGCCCACCGTGCCCAAGATCT
ATTCCTGGGCCACCTGGTCCGATAGGCCCAGAGGGTCCCAGAGGATTACCTGGTTTGCCA
GGAAGAGATGGTGTTCCTGGATTAGTGGGTGTCCCTGGACGTCCAGGTGTCAGAGGATTA
AAAGGCCTACCAGGAAGAAATGGGGAAAAAGGAGCCAAGGGTTTGGGTATCCTGGAGAA
CAAGGTCCTCCTGGTCCCCCAGGTCCAGAGGGCCCTCCTGGAATAAGCAAAGAAGGTCCT
CCAGGAGACCCAGGTCTCCCTGGCAAAGATGGAGACCATGGAAAACCTGGAATCCAAGGG
CAACCAGGCCCCCCAGGCATCTGCGACCCATCACTATGTTTTAGTGTAATTGCCAGAAGA
GATCCGTTCAGAAAAGGACCAAACTATAGTCTAGACGACAGCAGCCATCATCACCATCAC
CATAGCAGCGGC

The resulting three expression vectors were co-transfected into Drosophila S2 cells. The cells were cultured in the presence blasticidin to select blasticidin-resistant cells. Cell culture supernatants were collected and screened by Western Blot and ELISA for antibody activities against EGFR and CD3. It was found that cells of some clones stably expressed triple helix complexes. These triple helix complexes, like human minicollagen XXI, were resistant to heat and pepsin. More importantly, they specifically bound to both EGFR and CD3.

### Example 2

In this example, three fusion polypeptides: OKT3_scFv-Col, erb_scFv-Col, and erb_NSPD-scFv were generated.

### Selection of Phage Library

An erb phagemid containing an epidermal growth factor receptor extracellular domain (EGFR-ECD)-binding variable fragment (scFv) was isolated by screening a human single fold scFv phage display libraries (Tomlinson I + J; kindly provided by I. M. Tomlinson and G. Winter, MRC Laboratory of Molecular Biology, Cambridge, UK). Selections were performed using immunotubes (Maxisorp; Nunc, Roskilde, Denmark) coated with 10 µg of purified recombinant extracellular domain of EGF receptor (EGFR-ECD; Research Diagnostics, Inc.). Btocking, panning, washing, elution, and reamplification of eluted phage were carried out according to the manufacturer's protocol.

### Construction of Recombinant Plasmids

The cDNAs coding for the scFvs of erb were PCR amplified from the erb phagemid. A sequence encoding murine IgG2a anti-CD3 mAb OKT3 (Ortho Pharmaceutical Corporation) was obtained by reverse transcription product from OKT3 hybridoma (ATCC, CRL-8001). The cDNAs for the V_{L} and V_{H} of the OKT3 mAb were obtained by RT-PCR based on the published nucleotide sequence. The scFv PCR fusion of erb and OKT3 were generated by joining the V_{H} and V_{L} chains with a glycine-linker (GGGS)₃.

To generate scFv-Col, the coding region of scFv-Col included an N-terminal scFv nucleotide sequence and a C-terminal synthetic collagen scaffold gene coding for a peptide sequence of EPKSCDKTHTCPPCPRSIP(GPP)₁₀GICDPSLCFSVIA-RRDPFRKGPNY, which includes a hinge region of human IgG, a collageneous domain (underlined), and the NC1 domain of type XXI collagen. Shown below are the synthetic collagen scaffold polypeptide and cDNA sequences (SEQ ID NOs: 9 and 10, respectively).

SEQ ID NO: 9:
GluProLysSerCysAspLysThrHisThrCysProProCysProArgSerIleProGly
ProProGlyProProGlyProProGlyProProGlyProProGlyProProGlyProPro
GlyProProGlyProProGlyProProGlyIleCysAspProSerLeuCysPheSerVal
IleAlaArgArgAspProPheArgLysGlyProAsnTyr

SEQ ID NO: 10:
GAGCCCAAATCTTGTGACAAAACTCACACATGCCCACCGTGCCCAAGATCTATTCCTGGG
CCACCTGGTCCCCCAGGTCCTCCAGGACCCCCAGGGCCCCCAGGCCCCCCCGGCCGCCT
GGACCCCCAGGGCCACCAGGCCCCCCAGGCATCTGCGACCCATCACTATGTTTTAGTGTA
ATTGCCAGAAGAGATCCGTTCAGAAAAGGACCAAACTAT

This synthetic sequence (SEQ ID NO: 10) was prepared by overlapping PCR and the PCR product flanking with NotI and XhoI sites was cloned into the expression vector pSecTag2/Hygro (Invitrogen) at the same sites. The scFv of erb and OKT3 were then cloned in-frame to the above C-terminal collagen scaffold-containing construct at AscI and NotI sites to make the expression constructs of erb_scFv-Col and OKT3_scFv-Col, respectively.

Next, erb_NSPD-scFv was generated. The coding region of NSPD-scFv included the N-terminal 254 amino acids of human surfactant protein D (SPD), a member of the collectin family, and an scFv at the C-terminus. Shown below are N-terminal 254 amino acids of human surfactant protein D polypeptide and its cDNA sequence (SEQ ID NOs: 11 and 12, respectively).

SEQ ID NO: 11:
MetLeuLeuPheLeuLeuSerAlaLeuValLeuLeuThrGlnProLeuGlyTyrLeuGlu
AlaGluMetLysThrTyrSerHisArgThrMetProSerAlaCysThrLeuValMetCys
SerSerValGluSerGlyLeuProGlyArgAspGlyArgAspGlyArgGluGlyProArg
GlyGluLysGlyAspProGlyLeuProGlyAlaAlaGlyGlnAlaGlyMetProGlyGln
AlaGlyProValGlyProLysGlyAspAsnGlySerValGlyGluProGlyProLysGly
AspThrGlyProSerGlyProProGlyProProGlyValProGlyProAlaGlyArgGlu
GlyProLeuGlyLysGlnGlyAsnIleGlyProGlnGlyLysProGlyProLysGlyGlu
AlaGlyProLysGlyGluValGlyAlaProGlyMetGlnGlySerAlaGlyAlaArgGly
LeuAlaGlyProLysGlyGluArgGlyValProGlyGluArgGlyValProGlyAsnThr
GlyAlaAlaGlySerAlaGlyAlaMetGlyProGlnGlySerProGlyAlaArgGlyPro
ProGlyLeuLysGlyAspLysGlyIleProGlyAspLysGlyAlaLysGlyGluSerGly
LeuProAspValAlaSerLeuArgGlnGlnValGluAlaLeuGlnGlyGlnValGlnHis
LeuGlnAlaAlaPheSerGlnTyrLysLysValGluLeuPhe

SEQ ID NO: 12:
ATGCTGCTCTTCCTCCTCTCTGCACTGGTCCTGCTCACACAGCCCCTGGGCTACCTGGAA
GCAGAAATGAAGACCTACTCCCACAGAACAATGCCCAGTGCTTGCACCCTGGTCATGTGT
AGCTCAGTGGAGAGTGGCCTGCCTGGTCGCGATGGACGGGATGGGAGAGAGGGCCCTCGG
GGCGAGAAGGGGGACCCAGGTTTGCCAGGAGCTGCAGGGCAAGCAGGGATGCCTGGACAA
GCTGGCCCAGTTGGGCCCAAAGGGGACAATGGCTCTGTTGGAGAACCTGGACCAAAGGGA
GACACTGGGCCAAGTGGACCTCCAGGACCTCCCGGTGTGCCTGGTCCAGCTGGAAGAGAA
GGTCCCCTGGGGAAGCAGGGGAACATAGGACCTCAGGGCAAGCCAGGCCCAAAAGGAGAA
GCTGGGCCCAAAGGAGAAGTAGGTGCCCCAGGCATGCAGGGCTCGGCAGGGGCAAGAGGC
CTCGCAGGCCCTAAGGGAGAGCGAGGTGTCCCTGGTGAGCGTGGAGTCCCTGGAAACACA
GGGGCAGCAGGGTCTGCTGGAGCCATGGGTCCCCAGGGAAGTCCAGGTGCCAGGGGACCC
CCGGGATTGAAGGGGGACAAAGGCATTCCTGGAGACAAAGGAGCAAAGGAGAAAGTGGG

CTTCCAGATGTTGCTTCTCTGAGGCAGCAGGTTGAGGCCTTACAGGGACAAGTACAGCAC
CTCCAGGCTGCTTTCTCTCAGTATAAGAAAGTTGAGCTCTTC

The N-terminal SPD cDNA was cloned into the expression vector pSecTag2/Hygro (Invitrogen) at the NheI and AscI sites. The scFv of erb was then cloned in-frame to the above N-terminal SPD-containing construct at AscI and XhoI sites to make the expression construct of erb_NSPD-scFv.

Each open reading frame of erb_scFv-Col, erb_NSPD-scFv and OKT3_scFv-Col contained sequences encoding an N-terminal leader sequences and C-terminal myc epitope/polyhistidine tags for secretion, detecting, and purification purposes. Summarized in Table below are various recombinant proteins/antibodies encoded by the above-described expression constructs:

**Table 1. Overview of various antibody molecules used in this study**

| Antibody | Target | Type | Ab Origin |
|---|---|---|---|
| erb_scFv-Col | EGFR-ECD | CSA¹ | Human |
| erb_scFv-Fc | EGFR-ECD | scFv-Fc | Human |
| erb_scFv | EGFR-ECD | scFv | Human |
| erb_NSPD-scFv | EGFR-ECD | CSA | Human |
| OKT3_scFv-Col | CD3 | CSA | Murine |
| OKT3 | CD3 | IgG | Murine |

| | | | |
|---|---|---|---|
| ¹ Collagen scaffold antibody | | | |

### Expression and Purification of Antibodies

To generate the recombinant protein complexes/antibodies, the above-described constructs were transfected in mouse myeloma NS0 cells using Effectene (Qiagen) according to the manufacturer's instructions. After selection with Hygromycin (400 µg/ml) for 4 weeks, each stable clone was cultured in a shaker flask at an initial seeding density of 2 × 10⁵ cells/ml in a chemically-defined medium HyQCDM4NS0 (Hyclone) containing 2% of fetal bovine serum. The culture was maintained at 150 rpm for five days at 37 °C. Sodium ascorbate (80 µg/ml) was added to the culture media daily for those cells carrying expression constructs encoding proteins containing the above-mentioned antibody domains and the collagen scaffold domain, i.e., collagen scaffold antibodies (CSA).

To purify erb_scFv, erb_scFv-Fc, erb_scFv-Col, or OKT3_scFv-Col protein or protein complexes, around 2 L each of the filtered culture media were applied to a T-gel column (1.5 x 8 cm, Pierce) equilibrated with 50 mM Tris-HCl buffer, pH 8.0 at a flow rate of 60 ml/hour. After washing with the same buffer, the recombinant protein or protein complexes were eluted with 50 mM of sodium acetate buffer, pH4.0. Their UV absorbance was monitored at 280 nm and the peak fraction was applied onto a ZnSO₄-charged chelating Sepharose HighTrap column (1-ml in bed volume, GE Healthcare) equilibrated with 50 mM Tris-HCl buffer containing 0.5 M NaCl, pH 8.0 at a flow rate of 60 ml/hour. The column was first washed with 20 mM of imidazole and then the bound protein or protein complexes were eluted with 0.25 M of imidazole in the same buffer. The final preparation was dialyzed against 50 mM of Hepes buffer, pH 7.0.

Then, SDS-PAGE was carried out using either a 10% NuPAGE bis-Tris polyacrylamide gel with MOPS or a 7% SDS/Tris-acetate polyacrylamide gel with sodium acetate as running buffer (Invitrogen). Proteins were then stained with Coomassie brilliant blue R-250. Densities of protein bands were quantified by densitometry using ChemiImager 5500 (Alpha Innotech, San Leandro, CA) with Alpha EaseFC (v. 4.0; Alpha Innotech) software.

To examine the triple helical nature, purified erb_scFv-Col (1 mg/ml) was incubated at 37 °C in the absence or presence of 10 mM DTT for one hour. An aliquot from the DTT treated sample was further reacted with 50 mM N-ethyl-maleimide (NEM) for 30 minutes at an ambient temperature to permanently block free sulfhydryls and reformation oftrimers. An equal amount of protein from each sample was electrophoresed on a 7% SDS/Tris-acetate polyacrylamide gel with sodium acetate as the running buffer. The gel was stained with Coomassie blue. It was found that the purified CSAs were homotrimers or interchain disulfide-bonded heximers, which can be dissociated into two trimers under mild reducing conditions.

Thermal stability of the trimeric structure of erb_scFv-Col was examined. Purified erb_scFv-Col in 50 mM Tris-HCl (pH 8.0), containing 2 M urea was treated in the absence or presence of 10 mM tris(2-carboxyethyl)phosphine (TCEP) at an ambient temperature. The reduced samples were then alkylated with 50 mM ofNEM at an ambient temperature. Each sample with an equal amount of protein was heated for 10 minutes at 35, 45, 55, 65, 75, and 85°C before mixing the SDS-loading buffer. The samples were electrophoresed on a 10% SDS/Bis-Tris polyacrylamide gel with the MOPS buffer under non-reducing conditions. The gel was stained with Coomassie blue. The result indicated that erb_scFv-Col trimer exhibited high thermal stability. Indeed, after treated at and 65°C for 10 minutes, more than 50% of the trimers remained. It was also found that the trimeric structure of the collagenous domain of erb_scFv-Col was prolyl hydroxylated.

### Binding Studies

The binding kinetics of erb antibody variants to the EGFR-ECD were measured using a BIAcore X biosensor (BIACORE, Inc., Uppsala, Sweden) in the running buffer HBS-EP (10 mM HEPES, pH 7.4, 150 mM NaCl, 3 mM EDTA, 0.005% surfactant P20). Briefly, EGFR-ECD was immobilized onto a C1 sensor chip via amine coupling to a level of 1700 response units (RU) and purified antibodies with different concentrations were injected at a flow rate of 10 µl/min. The surface was regenerated by injection of 5 µl of 10 mM glycine-HCl, pH 3.5. Sensorgrams were obtained at each concentration and were evaluated using the program, BIA Evaluation 3.2. Binding data were fitted with a 1:1 Langmuir binding model to calculate the affinity constant, K*_{D}*, which was defined as the ratio of dissociation rate (k*_{diss}*)/association rate (k*ₐₛₛ*). The results were shown in Table 2 below.

**Table 2. Binding kinetics of various form of erb antibody to immobilized EGFR-ECD**

| ***Antibody*** | ***kₐₛₛ*/*10⁵*** | ***K_{diss}*/*10⁻⁴*** | ***K_{D}*** |
|---|---|---|---|
| | M⁻¹s⁻¹ | s⁻¹ | nM |
| erb_scFv_Col | 1.72 | 8.22 | 4.78 |
| erb_scFv-Fc | 0.909 | 94.4 | 104 |
| erb_scFv | 0.15 | 741 | 4960 |

As shown in Table 2, the binding affinity of erb_scFv_Col for EGFR-ECD is approximately 20- and 1000-fold stronger than the bivalent (erb_scFv-Fc) and monovalent (erb_scFv) mAb counterparts, respectively.

### Stability and Pharmacokinetic Assays

For serum stability assay, the stability of various forms of erb_scFv-Col, erb_scFv-Fc, or erb_scFv erb antibody was determined by incubating with human serum at 37 °C. The amount of active anti-EGFR remaining after different periods of incubation times was measured by quantitative ELISA. The ELISA was conducted by employing the recombinant EGFR-ECD (as capture reagent) and anti-c-myc mAb (9E10, Sigma Chemical Co.), followed by an HRP-conjugated affinity-purified polyclonal goat antimouse IgG and chemiluminescent substrates (Pierce Biotechnology, Inc.). For pharmacokinetic assay, three BALB/c nude mice were used to analyze erb_scFv-Col clearance. Briefly, following a pre-bleed, each mouse was injected subcutaneously (s.c.) with 25 µg (2 mg/kilogram of body weight) of erb_scFv-Col. During the next 70 hours, periodic blood samples were collected and evaluated for their content of erb_scFv-Col by ELISA. It was found the proteins were quite stable.

### T Cell Proliferation Assay and Mixed Lymphocyte Reaction (MLR)

5-bromo-2'-deoxyuridine (BrdU) cell proliferation assay was performed. Briefly, human peripheral blood mononuclear cells (PBMCs) were plated in a black 96-well flat bottom tissue culture plate at 2 × 10⁵ cells/well in 100 µl RPMI-1640 medium with 10% FBS at 37°C in the presence of 10-fold serial dilution of OKT3 (eBioscience, Inc.) or OKT3_scFv-Col for 66 hours. The cells were then pulsed with 10 µM of BrdU for 6 hours. After removing the culture medium, the cells were fixed and DNA was denatured in one step with FixDenat. Afterward, the cells were incubated with a peroxidase labeled anti-BrdU antibody (anti-BrdU POD, Fab fragments) for 1.5 hours at room temperature. Chemiluminescence detection and quantification was performed using a microplate-luminometer (Hidex, CHAMELEON detection platform, Finland).

T cell proliferation and immunosuppression in the one-way mixed lymphocyte reaction was assessed as follows. Human PBMCs were obtained from two healthy donors (stimulator and responder). Stimulator or responder cells were treated with 25 µg/ml of mitomycin C (Sigma-Aldrich) in a complete medium (RPMI 1640 supplemented with 10% human AB serum, 2 mM glutamine, 50 nM 2-mercaptoethanol, and 100 units/ml each of penicillin and streptomycin) for 30 minutes in humidified air containing 5% CO2 at 37°C, followed by three washes in RPMI 1640 medium. Responder cells were cultured alone or mixed with mitomycin C treated stimulator or mitomycin C responder cells at 1:1 ratio at 2×10⁵ cells/well in 200 µl of complete medium. Purified OKT3_scFv-Col or OKT3 was added at different concentrations to cultures immediately after responder cell plating. After 5 days, cultured cells were pulsed with 10 µM of BrdU and harvested 24 h later. Cell proliferation assay was then performed in the manner described above.

It was found that OKT3_scFv_Col is more effective at immunosuppressing T cell proliferation while exhibiting negligible mitogenic activity in stimulating T cell proliferation.

### Cytokine Measurement

Human PBMCs were plated at 2 × 10⁵ cells/well in 0.1 ml RPMI-1640 medium with 10% FBS at 37°C in the presence of 10-fold serial dilution of OKT3 or OKT3_scFv-Col. The supernatants were harvested at different time points and multiple cytokines were measured using a human cytokine immunoassay kit (eBioscience, Inc.). The results indicated that administration of OKT3_scFv_Col causes negligible cytokine releasing as compared with murine OKT3 mAb.

### Antibody Displacement Assays

All of the following procedures were conducted at 4°C. Human T cells were suspended in an FCM buffer (phosphate-buffered saline with 2 % FBS and 0.1% sodium azide) at a density of 1 × 10⁶ cells/ml. The cells were treated with mouse total IgGs (2 µg/ml, Jackson ImmunoResearch Laboratories) for 30 minutes and were then incubated with a serial dilution of OKT3_seFv-Col or OKT3 antibody for 1 hour. A fixed, saturating amount (determined by flow cytometry) of FITC-conjugated OKT3 (0.25 µg /ml, purchased from eBioscience, Inc.) was added directly. After incubation for 1 hour, the cells were washed with the FCM buffer and analyzed for immunofluorescence by flow cytometry on a FACScan (Becton Dickinson, San Jose, CA). The data were presented as percent inhibition of maximal fluorescence intensity, which is defined as the mean fluorescence intensity obtained by staining T cells with OKT3-FITC in the absence of blocking antibodies.

The result indicated that OKT3_scFv_Col binds to human CD3+ T cells stronger than the native murine OKT3 mAb.

Protein concentration was determined by the Bradford assay (Coomassie plus reagent, from Pierce Biotechnology, Inc.) using human IgGs as the standard. For amino acid analysis, purified erb_scFv-Col was dialyzed against 50 mM acetic acid, hydrolyzed in 6 N of HCl at 110°C for 24 hours and subjected to amino acid analysis in a Waters Pico·Tag^{®} system.

These results demonstrate that collagen scaffold antibody should be an ideal structure for therapeutic antibody design both in antitumor and immunomodulatory applications.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

The present invention also provides:
1. A recombinant protein complex comprising
   a first fusion polypeptide chain containing a first scaffold domain and a first heterologous domain fused to one end of the first scaffold domain;
   a second fusion polypeptide chain containing a second scaffold domain; and
   a third fusion polypeptide chain containing a third scaffold domain; wherein the first, second, and third scaffold domains are aligned to form a triple helix coil.
2. The protein complex of embodiment 1, wherein the heterologous domain contains a binding domain.
3. The protein complex of embodiment 2, wherein the binding domain contains a ligand binding domain, a ligand, a receptor, an affinity tag, or a proteoglycan.
4. The protein complex of embodiment 2, wherein the binding domain contains one or more complementary-determining regions of an immunoglobulin.
5. The protein complex of embodiment 4, wherein the binding domain contains the sequence of an antigen-binding fragment.
6. The protein complex of embodiment 5, wherein the antigen-binding fragment specifically binds to CD3, or EGFR.
7. The protein complex of embodiment 5, wherein the antigen-binding fragment includes the sequence of a single chain antibody.
8. The protein complex of embodiment 1, wherein the first fusion polypeptide chain further contains a second heterologous domain fused to the other end of the first scaffold domain.
9. The protein complex of embodiment 8, wherein the first heterologous domain contains the sequence of a first single chain antibody that specifically binds to CD3.
10. The protein complex of embodiment 8, wherein the second heterologous domain contains the sequence of a second single chain antibody that specifically binds EGFR.
11. The protein complex of embodiment 8, wherein the second fusion polypeptide chain contains a third heterologous domain fused to one end of the second scaffold domain.
12. The protein complex of embodiment 11, wherein the second fusion polypeptide chain further contains a fourth heterologous domain fused to the other end of the second scaffold domain.
13. The protein complex of embodiment 12, wherein the third fusion polypeptide chain contains a fifth heterologous domain fused to one end of the third scaffold domain.
14. The protein complex of embodiment 13, wherein the third fusion polypeptide chain contains a sixth heterologous domain fused to the other end of the third scaffold domain.
15. The protein complex of embodiment 1, wherein the first, second, or third scaffold domain contains one or more triple helix repeats, each repeat containing a sequence of the following formula: (X1-X2-X3)n, wherein X1 is a Gly residue, X2 or X3 is any amino acid residue, and n is 5 or greater.
16. The protein complex of embodiment 15, wherein the first, second, or third scaffold domain contains one or more triple helix repeats of Clq, collectin, or a collagen polypeptide chain.
17. The protein complex of embodiment 15, wherein X3 is a proline or hydroxyproline residue.
18. The protein complex of embodiment 15, wherein each repeat containing a sequence of (GPP)₁₀.
19. The protein complex of embodiment 1, wherein the heterologous domain contains the sequence of an enzymatic domain or a fluorescent protein.
20. The protein complex of embodiment 1, wherein the first, second, and third fusion polypeptides are substantially identical.
21. A recombinant fusion polypeptide comprising a scaffold domain for forming a triple helix coil and a first heterologous domain fused to one end of the scaffold domain.
22. The fusion polypeptide of embodiment 21, wherein the scaffold domain contains one or more triple helix repeats, each repeat containing a sequence of the following formula: (X1-X2-X3)n, wherein X1 is a Gly residue, X2 or X3 is any amino acid residue, and n is 5 or greater.
23. The fusion polypeptide of embodiment 22, wherein X3 is a proline or hydroxyproline residue.
24. The fusion polypeptide of embodiment 21 further comprises a second heterologous domain fused to the other end of the scaffold domain.
25. The fusion polypeptide of embodiment 21, wherein the scaffold domain contains one or more triple helix repeats of Clq, collectin, or a collagen polypeptide chain.
26. The fusion polypeptide of embodiment 21, wherein the heterologous domain contains a ligand binding domain, a ligand, a receptor, or a polysaccharide.
27. The fusion polypeptide of embodiment 26, wherein the heterologous domain is obtained by phage display screening.
28. An isolated nucleic acid comprising a sequence encoding a fusion polypeptide of embodiment 21, or a complement thereof.
29. A host cell comprising a nucleic acid of embodiment 28.
30. The host cell of embodiment 29, wherein the cell is a mammalian or an insect cell.
31. The host cell of embodiment 30, wherein the mammalian cell is a mouse myeloma NS0 cell.
32. An expression vector comprising a nucleic acid of embodiment 28.
33. A method of producing a fusion polypeptide, comprising culturing the host cell of embodiment 29 in a medium under conditions permitting expression of a polypeptide encoded by the nucleic acid, and purifying the polypeptide from the cultured cell or the medium of the cell.
34. A method of producing a protein complex of embodiment 1, comprising
   culturing a host cell containing a first nucleic acid encoding a first fusion polypeptide chain containing a first scaffold domain and a first heterologous domain fused to one end of the first scaffold domain, a second nucleic acid encoding a second fusion polypeptide chain containing a second scaffold domain, and a third nucleic acid encoding a third fusion polypeptide chain containing a third scaffold domain in a medium under a condition permitting expression of polypeptides encoded by the three nucleic acids and formation of a triple helix coil therebetween; and
   purifying the protein complex from the cultured cell or the medium of the cell.
35. A method according to embodiment 34, wherein the host cell is a eukaryotic cell containing an enzymatic activity that hydroxylates a proline residue.

## Claims

1. A trimeric protein complex comprising three polypeptides, wherein each polypeptide comprises:
(a) a triple helix coil scaffold domain comprising one or more triple helix repeats, wherein each repeat is a sequence of the following formula: (X₁-X₂-X₃)ₙ, wherein X₁ is a Gly residue, X₂ and X₃ are any amino acid residues, and n is 5 or greater;
(b) a first heterologous domain in-frame fused to one end of the scaffold domain, wherein the first heterologous domain is a first antibody domain comprising:
(i) SEQ ID NO:1;
(ii) SEQ ID NO:5;
(iii) a sequence encoded by a nucleic acid comprising SEQ ID NO:2; or
(iv) a sequence encoded by a nucleic acid comprising SEQ ID NO:6;
(c) optionally a second heterologous domain in-frame fused to the other end of the scaffold domain, wherein the second heterologous domain is a binding domain selected from the group consisting of a second antibody domain, a ligand binding domain, a ligand, a receptor, and a proteoglycan, or which is a fluorescent protein or an enzymatic domain;
wherein the triple helix coil scaffold domains of the three polypeptides interact with each other to form a trimeric protein complex.

2. The protein complex of claim 1, wherein X₂ and X₃ are any proline or hydroxyproline residue.

3. The protein complex of any of claims 1 or 2, wherein one or more proline residues is replaced by hydroxyproline.

4. The protein complex of any of claims 1-3, wherein the first antibody domain is in-frame fused to the amino terminus of the scaffold domain.

5. The protein complex of any of claims 1-3, wherein the first antibody domain is in-frame fused to the carboxy terminus of the scaffold domain.

6. The protein complex of any of claims 1-5, wherein the second antibody domain comprises a single chain antibody.

7. The protein complex of any of claims 1-6, wherein each polypeptide has a second heterologous domain consisting of a second antibody domain.

8. The protein complex of any of claims 1-7, wherein the scaffold domain comprises 10 G-P-P repeats.

9. The protein complex of any of claims 1-5, wherein at least one of the polypeptides has a second heterologous domain consisting of an enzymatic domain or a fluorescent protein.

10. The protein complex of any of claims 1-9, wherein the three polypeptides are substantially identical.

11. An isolated nucleic acid comprising a sequence encoding a polypeptide of any of claims 1-10.

12. An expression vector comprising a nucleic acid of claim 11.

13. A host cell comprising a nucleic acid of claim 11 or an expression vector of claim 12.

14. The host cell of claim 13, wherein the cell is a mammalian or an insect cell.

15. The host cell of claim 14, wherein the mammalian cell is a mouse myeloma NS0 cell.

16. A pharmaceutical composition comprising the protein complex of any one of claims 1-10.

17. A method of producing a trimeric protein complex of claim 1, comprising culturing a host cell containing a first nucleic acid encoding a first polypeptide chain containing a first triple helix scaffold domain and a first heterologous domain fused to one end of the first scaffold domain, a second nucleic acid encoding a second polypeptide chain containing a second triple helix scaffold domain, and a third nucleic acid encoding a third polypeptide chain containing a third triple helix scaffold domain in a medium under a condition permitting expression of polypeptides encoded by the three nucleic acids and formation of a triple helix coil therebetween; and purifying the protein complex from the cultured cell or the medium of cell.

18. A method according to claim 17, wherein the host cell is a eukaryotic cell containing an enzymatic activity that hydroxylates a proline residue.
